(19) Europäisches Patentamt
European Patent Office
Office européen des brevets

(11) Numéro de publication: **0 047 205**
**B1**

(12) # FASCICULE DE BREVET EUROPEEN

(45) Date de publication du fascicule du brevet: **25.04.84**

(51) Int. Cl.³: **C 09 B 1/48**, C 09 B 5/28, C 07 C 103/82, C 07 C 97/24

(21) Numéro de dépôt: **81401323.1**

(22) Date de dépôt: **20.08.81**

(54) **Procédé de préparation d'alpha-benzamido alpha'-halogéno dianthrimides-1,1', nouveaux alpha-benzamido halogéno-5' ou -8' dianthrimides-1,1' et leur application comme intermédiaires de colorants de cuve.**

(30) Priorité: **02.09.80 FR 8018908**

(43) Date de publication de la demande: **10.03.82 Bulletin 82/10**

(45) Mention de la délivrance du brevet: **25.04.84 Bulletin 84/17**

(84) Etats contractants désignés: **BE CH DE FR GB IT LI**

(56) Documents cités:
**FR - A - 1 212 316**

**INDIAN JOURNAL OF CHEMISTRY, vol. 14B, no. 3, mars 1976, Londres, GB S.V. SUNTHANKAR et al.: "Cyclization of substituted anthrimides & 4-arylamino-1-benzamidoanthraquinones", pages 171-175**
**CHEMICAL ABSTRACTS, vol. 51, no. 6, 25 mars 1957, colonne 4198e Columbus, Ohio, US R.L. GROB et al.: "Determination of trace quantities of boron with two new colorimetric reagents"**

(73) Titulaire: **I.C.I. FRANCOLOR S.A., 8 Avenue Reaumur Boîte Postale 207, F-92142 Clamart Cedex (FR)**

(72) Inventeur: **Devic, Michel, 74, chemin du Fort Saint-Irénée, F-69005 Lyon (FR)**
Inventeur: **Nicaise, Jean-Claude René, 86, rue Boilet, F-60700 Pont Sainte Maxence (FR)**
Inventeur: **Pailler, Jacques, 9 Squaire Jean Du Bellay Les Trois Clairières, F-60270 Gouvieux (FR)**

(74) Mandataire: **Pugsley, Roger Graham et al, IMPERIAL CHEMICAL INDUSTRIES PLC Legal Department: Patents PO Box 6, Welwyn Garden City Herts, AL7 1HD (GB)**

**EP 0 047 205 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Procédé de préparation d'α-benzamido α'-halogéno dianthrimides-1,1', nouveaux α-benzamido halogéno-5' ou -8' dianthrimides-1,1' et leur application comme intermédiaires de colorants de cuve

La présente invention, faite dans les services de la demanderesse, concerne le domaine des intermédiaires pour colorants de cuve de la série du dianthrimide-1,1', c'est-à-dire de la dianthraquinonyl-1,1' amine. Plus précisément, la présente invention a pour objet la préparation d'α-benzamido d'α-halogéno dianthrimides-1,1' et leur utilisation comme précurseurs de colorants de cuve du type α,α'-dibenzamido diphtaloylcarbazole.

Les dianthrimides concernés par l'invention peuvent être représentés par la formule générale:

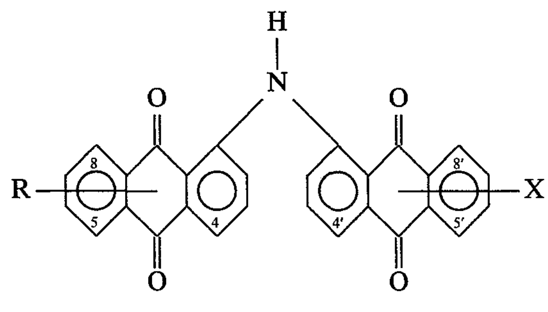

(I)

dans laquelle R représente un groupe benzamido éventuellement substitué, fixé en position 4, 5 ou 8 et X représente un atome d'halogène (de préférence brome ou chlore) fixé en position 4', 5' ou 8'. Comme substituants du groupe benzamido, on peut citer les atomes d'halogène et les groupes alkyle, alcoxy, nitro, butadiène-1,3 ylène (R=naphtalènecarboxamido) ou pthaloyle (R=anthraquinonecarboxamido). Les α-benzamido halogéno-5' ou -8' dianthrimides compris dans la formule (I) sont des produits nouveaux et, en tant que tels, font partie de la présente invention.

Dans Indian Journal of Chemistry, Vol. 14B, N° 3, pages 171—175, SUNTHANKAR et al. ont déjà décrit le benzamido-4 chloro-4' dianthrimide-1,1' qu'ils ont préparé par condensation de la chloro-1 benzamido-4 anthraquinone avec l'amino-1 chloro-4 anthraquinone.

Parmi les colorants de cuve du type α,α'-dibenzamido diphtaloylcarbazole, ceux connus au Colour Index sous les noms VAT BROWN 3 (C. I. 69015) et VAT ORANGE 15 (C. I. 69025) sont jusqu'à présent obtenus par condensation de la benzamido-1 chloro-5 anthraquinone avec respectivement la benzamido-1 amino-4 anthraquinone et la benzamido-1 amino-5 anthraquinone, suivie d'une cyclisation. Cependant, la synthèse de la benzamido-1 chloro-5 anthraquinone selon les étapes suivantes:

dichloro-1,5 anthraquinone

↓

p-toluènesulfonamido-1 chloro-5 anthraquinone

↓

amino-1 chloro-5 anthraquinone

↓

benzamido-1 chloro-5 anthraquinone

est particulièrement longue et génératrice d'effluents à taux de pollution élevé. Quant au colorant VAT BLACK 27 (C. I. 69005), le procédé le plus couramment utilisé pour sa préparation consiste en une condensation de l'amino-1 anthraquinone et de la chloro-1 anthraquinone en dianthrimide-1,1', suivie par une dinitration en positions 4 et 4', réduction, dibenzoylation et cyclisation.

Il a maintenant été trouvé qu'il est possible d'obtenir ces colorants de cuve et autres analogues par condensation d'une dihalogéno-1,4 (ou 1,5 ou 1,8) anthraquinone et de l'amino-1 benzamido-4 (ou 5 ou 8) anthraquinone en α-benzamido α'-halogéno dianthrimide-1,1' de formule (I), suivie par une benzamidation et une cyclisation en l'α,α'-dibenzamido diphtaloylcarbazole correspondant. Cette nouvelle technique passant par les composés de formule (I) présente les avantages suivants:

— accès aux bases de colorants de cuve, c'est-à-dire aux α,α'-dibenzamido dianthrimides-1,1', en deux étapes seulement à partir d'une dihalogéno-1,4 (ou 1,5 ou 1,8) anthraquinone au lieu de quatre étapes selon les procédés antérieurs ci-dessus exposés, d'où une amélioration très

sensible de la productivité et une diminution des coûts de fabrication;
— absence des rejets d'acide p-toluènesulfonique provenant de l'hydrolyse de la p-toluènesulfonamido-1 chloro-5 anthraquinone en chloro-1 amino-5 anthraquinone;
— absence d'autres rejets grâce à la possibilité de récupérer et de recycler les produits n'ayant pas réagi lors de la condensation ou de la benzamidation.

D'autre part, lorsque la condensation de la dihalogéno-1,4 (ou 1,5 ou 1,8) anthraquinone et de l'amino-1 benzamido-4 (ou 5 ou 8) anthraquinone est effectuée en milieu organique, la benzamidation de l'α-benzamido α'-halogéno dianthrimide-1,1' peut être réalisée dans le même milieu, si bien qu'à partir d'une dihalogéno-1,4 (ou 1,5 ou 1,8) anthraquinone on accède dans ce cas à la base de colorant de cuve en une seule opération.

Comme exposé ci-dessus, les α-benzamido α'-halogéno dianthrimides de formule (I) sont préparés selon l'invention par condensation d'une amino-1 benzamido-4 (ou 5 ou 8) anthraquinone (II) et d'une dihalogéno-1,4 (ou 1,5 ou 1,8) anthraquinone (III) telle que, par exemple, la dibromo-1,5 anthraquinone, la dichloro-1,4 anthraquinone, la dichloro-1,5 anthraquinone ou la dichloro-1,8 anthraquinone, selon le schéma réactionnel suivant:

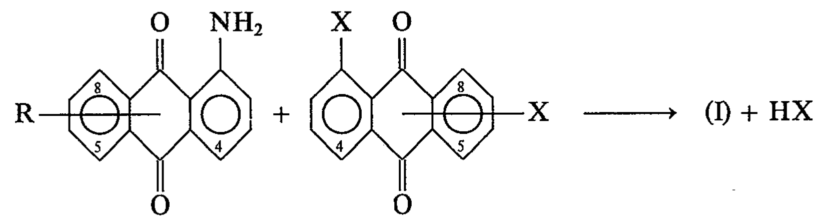

(II) (III)

Cette réaction est effectuée dans l'eau ou au sein d'un solvant organique en présence d'un catalyseur à base de cuivre et d'un agent capteur d'acide, à une température comprise entre 130 et 220° C (de préférence entre 140 et 170° C en milieu organique ou entre 185 et 220° C en milieu aqueux), le rapport molaire: dihalogéno-anthraquinone (III)/amino-benzamido-anthraquinone (II) étant supérieur à 1 et de préférence au moins égal à 1,35 lorsqu'on opère en milieu organique.

Comme catalyseur à base de cuivre, on peut utiliser le cuivre lui-même ou ses sels, de préférence l'acétate cuivrique préparé in situ ou non. La proportion de catalyseur à mettre en oeuvre est comprise entre 0,01 à 0,3 mole (de préférence entre 0,05 et 0,28 mole) de catalyseur par mole d'amino-1 benzamido-4 (ou 5 ou 8) anthraquinone (II).

L'agent capteur d'acide peut être choisi parmi les sels alcalins ou alcalino-terreux (par exemple carbonate de sodium, de potassium ou de magnésium, bicarbonate de sodium ou de potassium, acétate de sodium) et les oxydes alcalino-terreux (par exemple l'oxyde de magnésium); on préfère employer le bicarbonate de sodium ou de potassium. Cet agent capteur d'acide est introduit en excès par rapport à l'amino-1 benzamido-4 (ou 5 ou 8) anthraquinone selon un rapport molaire compris entre 1,5 et 7,5.

La réaction est de préférence réalisée au sein d'un solvant organique permettant d'opérer en milieu homogène tel que, par exemple, le nitro-2 toluène, les polyhalogénobenzènes et surtout le nitrobenzène et le naphtalène. Pour minimiser la formation parasite d'α,α''-dibenzamido trianthrimide de formule:

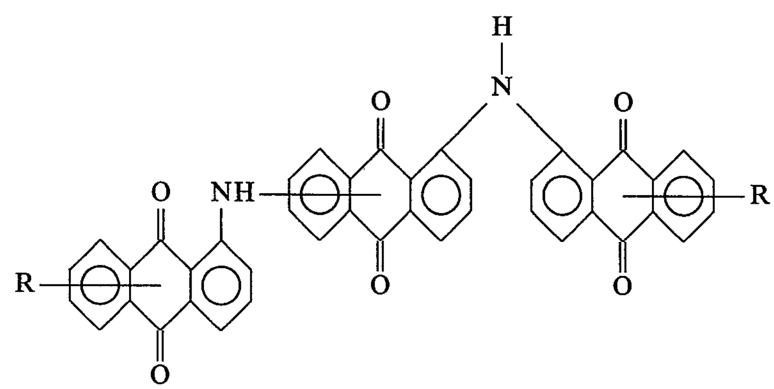

(IV)

provenant de la dicondensation de deux moles d'aminobenzamido-anthraquinone (II) avec une mole de dihalogéno-anthraquinone (III), il s'est révélé particulièrement avantageux d'opérer de telle sorte que la concentration molaire de dihalogéno-anthraquinone (III) dissoute dans le milieu réactionnel soit très supérieure à celle de l'α-benzamido α'-halogéno dianthrimide-1,1' (I) dissous. On opère donc de

préférence dans le volume minimum de solvant, de façon à limiter la quantité d'α-benzamido α'-halogéno dianthrimide-1,1' dissous à sa valeur de saturation dans les conditions réactionnelles; ainsi, par exemple, à 160°C dans le nitrobenzène en présence de dichloro-1,5 anthraquinone, la saturation du benzamido-4 chloro-5' dianthrimide-1,1' correspond à une concentration molaire inférieure à 0,05 mole/litre alors que la concentration de saturation de la dichloro-1,5 anthraquinone dans les mêmes conditions est supérieure à 2 moles/litre. La concentration molaire initiale en amino-benzamido-anthraquinone (II) sera de préférence supérieure à 0,1 mole/litre.

La benzamidation des α-benzamido α'-halogéno dianthrimides de formule (I) en α,α'-dibenzamido dianthrimides et la cyclisation de ces derniers en colorants de cuve du type α,α'-dibenzamido diphtaloylcarbazole peuvent être effectuées suivant les méthodes habituelles de benzamidation et de cyclisation.

Les exemples suivants illustrent l'invention; les indications de pourcentages s'entendent en poids.

## Exemple 1

Dans un réacteur équipé d'un agitateur à hélice, d'un réfrigérant descendant et d'un dispositif de régulation de la température, on introduit 120,4 g de nitrobenzène, 20 g de benzamido-1 amino-4 anthraquinone à 85,5% de pureté (0,05 mole), 27,45 g de dibromo-1,5 anthraquinone à 96,8% (0,0726 mole), 12,6 g de bicarbonate de sodium (0,15 mole) et 0,8 g d'acétate de cuivre monohydraté [$Cu(CH_3COO)_2$, $H_2O$] (0,004 mole). On chauffe sous agitation à 140°C, maintient à cette température durant 6 heures, puis à 145°C pendant 5 heures jusqu'à disparition de la benzamido-1 amino-4 anthraquinone en chromatographie en couche mince (CCM).

Après dilution par 180,6 g de nitrobenzène, on chauffe le mélange réactionnel à 145°C durant une heure, puis le porte à 160°C et l'essore à cette température sur filtre à plaque en verre fritté de porosité 2. Le gâteau de filtration est ensuite lavé deux fois avec chaque fois 120,4 g de nitrobenzène préalablement chauffé à 160°C, puis lavé au méthanol et à l'eau distillée jusqu'à neutralité.

Après séchage, on obtient 25,96 g d'un produit dont la teneur en benzamido-4 bromo-5' dianthrimide-1,1'

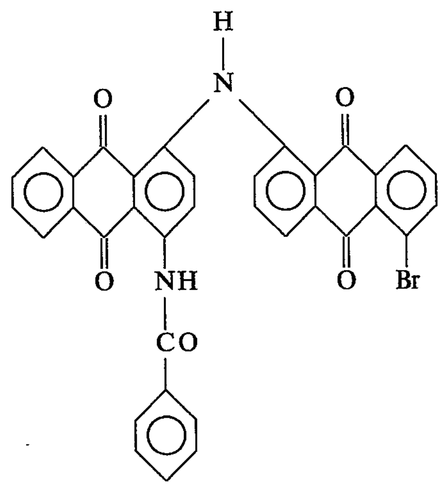

déterminée par chromatographie liquide haute pression (HPLC) est de 85%, soit un rendement chimique de 70,5% par rapport à la benzamido-1 amino-4 anthraquinone engagée.

Pour recristalliser ce produit, on le solubilise dans 3 120 g de nitrobenzène à 200°C, filtre sur filtre à plaque en verre fritté de porosité 3 et refroidit le filtrat à 25°C; après essorage du précipité sur filtre à plaque en verre fritté de porosité 2, lavage au méthanol et séchage, on obtient 21,3 g de benzamido-4 bromo-5' dianthrimide-1,1' à 96,8% de pureté (déterminée par HPLC) présentant les caractéristiques suivantes:

Analyse élémentaire (%):

Calculé pour $C_{35}H_{19}BrN_2O_5$

| | | | | |
|---|---|---|---|---|
| C 66,98 | H 3,03 | Br 12,76 | N 4,46 | O 12,76 |

Trouvé

| | | | | |
|---|---|---|---|---|
| C 66,62 | H 3,24 | Br 12,30 | N 4,49 | O 12,93 |

Spectre de masse:

$M^+$ à m/e = 626 (autres pics à 608, 592, 564, 548, 530 et 514).

Exemple 2

Dans 90,3 g de nitrobenzène on introduit 18 g de benzamido-1 amino-5 anthraquinone à 92% (0,0484 mole) et 27,45 g de dibromo-1,5 anthraquinone à 96,8% (0,0726 mole), puis chauffe sous agitation à 120° C et ajoute 12,6 g de bicarbonate de sodium (0,15 mole) et 0,8 g d'acétate cuivrique monohydraté (0,004 mole). On porte à 140° C et maintient à cette température pendant 8 heures jusqu'à disparition de la benzamido-1 amino-5 anthraquinone en CCM.

En opérant ensuite comme à l'exemple 1, on obtient 23,4 g de benzamido-5 bromo-5' dianthrimide-1,1' brut

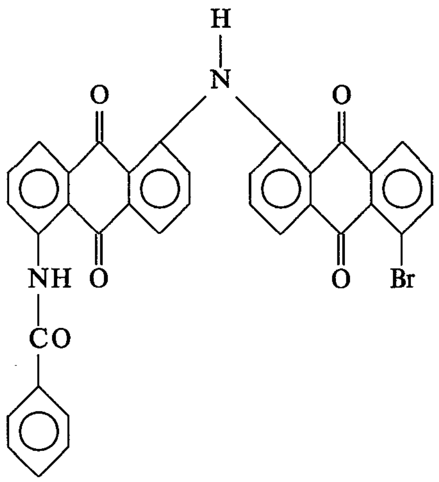

qui, après recristallisation, fournit 14,05 g d'un produit présentant les caractéristiques suivantes:

Analyse élémentaire (%):

Calculé pour $C_{35}H_{19}BrN_2O_5$

C 66,98 H 3,03 Br 12,76 N 4,46 O 12,76

Trouvé

C 66,84 H 3,24 Br 12,20 N 4,72 O 12,99

Spectre de masse:

$M^+$ à m/e = 626 (autres pics à 564 et 548).

Exemple 3

Dans 90,3 g de nitrobenzène on introduit à 120° C 17,1 g de benzamido-1 amino-4 anthraquinone à 99% (0,0495 mole), 28,75 g de dichloro-1,4 anthraquinone à 96% (0,0996 mole), 12,6 g de bicarbonate de sodium (0,15 mole) et 0,7 g d'acétate cuivrique monohydraté (0,0035 mole). On chauffe ensuite à 160° C pendant 6 heures jusqu'à disparition de la benzamido-1 amino-4 anthraquinone en CCM.

Après isolement selon le mode opératoire de l'exemple 1, on obtient 24,75 g d'un produit dont la teneur en benzamido-4 chloro-4' dianthrimide-1,1'

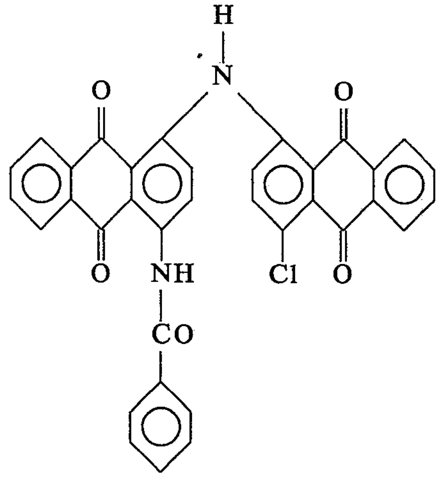

déterminée par HPLC est de 93,6%, soit un rendement chimique de 80,35% par rapport à la benzamido-1 amino-4 anthraquinone engagée.

Après recristallisation de ce produit brut comme à l'exemple 1, on obtient 18,1 g de benzamido-4 chloro-4' dianthrimide-1,1' à 99,8% de pureté (déterminée par HPLC) présentant les caractéristiques suivantes:

Analyse élémentaire (%):

Calculé pour $C_{35}H_{19}ClN_2O_5$

| | | | | |
|---|---|---|---|---|
| C 72,10 | H 3,26 | Cl 6,09 | N 4,80 | O 13,73 |

Trouvé

| | | | | |
|---|---|---|---|---|
| C 71,72 | H 3,40 | Cl 5,90 | N 4,57 | O 13,74 |

Spectre de masse:

$M^+$ à m/e = 582 (autre pic à 548).

## Exemple 4

Dans 361,2 g de nitrobenzène, on introduit 68,4 g de benzamido-1 amino-4 anthraquinone à 85,5% (0,171 mole), 115 g de dichloro-1,5 anthraquinone à 90,5% (0,376 mole), 50,4 g de bicarbonate de sodium (0,6 mole) et 2,8 g d'acétate cuivrique monohydraté (0,014 mole). On chauffe ensuite pendant 5 heures à 160° C jusqu'à disparition de la benzamido-1 amino-4 anthraquinone en CCM.

Après dilution avec 722,4 g de nitrobenzène, on chauffe le mélange réactionnel à 145° C pendant une heure, puis le porte à 160° C et l'essore à cette température sur filtre à plaque en verre fritté de porosité 2. Le gâteau de filtration est ensuite lavé deux fois avec deux fractions de 482 g de nitrobenzène à 160° C, puis lavé au méthanol et à l'eau distillée jusqu'à neutralité. Après séchage, on obtient 90 g d'un produit dont la teneur en benzamido-4 chloro-5' dianthrimide-1,1'

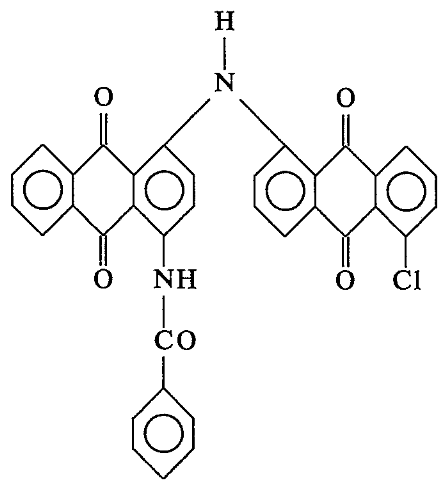

déterminée par HPLC est de 76%, soit un rendement chimique de 68,7%.

Aprés recristallisation de ce produit brut selon le mode opératoire de l'exemple 1, on obtient 64,8 g de benzamido-4 chloro-5' dianthrimide-1,1' à 99% de pureté (HPLC), présentant les caractéristiques suivantes:

Analyse élémentaire (%):

Calculé pour $C_{35}H_{19}ClN_2O_5$

| | | | | |
|---|---|---|---|---|
| C 72,10 | H 3,26 | Cl 6,09 | N 4,80 | O 13,73 |

Trouvé

| | | | | |
|---|---|---|---|---|
| C 70,92 | H 3,35 | Cl 5,60 | N 4,49 | O 14,77 |

Spectre de masse:

$M^+$ à m/e = 582 (autre pic à 478).

## Exemple 5

Dans 400 g de naphtalène à 120° C on introduit 78,5 g de dichloro-1,5 anthraquinone à 90,5% (0,256 mole), 68,4 g de benzamido-1 amino-4 anthraquinone à 85,5% (0,171 mole), 50,4 g de bicarbonate de

sodium (0,6 mole) et 2,8 g d'acétate cuivrique monohydraté (0,014 mole). On chauffe ensuite pendant 8 heures à 160° C jusqu'à disparition en CCM de la benzamido-1 amino-4 anthraquinone. Après dilution par 456,75 g de dichloro-1,2 benzène et chauffage à 160° C pendant une heure, la masse réactionnelle est essorée sur filtre à plaque en verre fritté de porosité n° 2. Le gâteau de filtration est ensuite lavé deux fois avec chaque fois 293,6 g de dichloro-1,2 benzène à 170° C, puis lavé au méthanol et à l'eau jusqu'à neutralité. Après séchage, on obtient 142,5 g de benzamido-4 chloro-5' dianthrimide-1,1' à 61% de pureté, soit un rendement chimique de 87,27% par rapport à la benzamido-1 amino-4 anthraquinone introduite.

Les caractéristiques analytiques de ce produit, après recristallisation, sont identiques à celles du produit de l'exemple 4.

## Exemple 6

Dans un mélange, maintenu à 160° C, de 228,75 g de nitrobenzène, 113,57 g de dichloro-1,5 anthraquinone à 99% (0,4059 mole), 18 g de benzamido-4 chloro-5' dianthrimide-1,1' à 76% de pureté (utilisé comme support de nucléation), 20,1 g de bicarbonate de sodium (0,239 mole) et 1,12 g d'acétate cuivrique monohydraté (0,00561 mole), on introduit en 2 heures une solution à 160° C de 23,39 g de benzamido-1 amino-4 anthraquinone à 99% (0,0677 mole) dans 349,1 g de nitrobenzène. On maintient pendant 2 heures à 160° C jusqu'à disparition de la benzamido-1 amino-4 anthraquinone en CCM. Après essorage selon les conditions de l'exemple 1, lavage par deux fractions de 135,4 g de nitrobenzène à 160° C, au méthanol et à l'eau distillée jusqu'à neutralité et séchage, on obtient 42,3 g de benzamido-4 chloro-5' dianthrimide-1,1' à 92,3% de pureté (HPLC), soit un rendement chimique de 64,3% par rapport à la benzamido-1 amino-4 anthraquinone introduite.

Les caractéristiques analytiques du produit recristallisé sont identiques à celles du produit de l'exemple 4.

## Exemple 7

Dans 120,4 g de nitrobenzène on introduit 17,1 g de benzamido-1 amino-4 anthraquinone à 85,5% (0,0427 mole), 28,75 g de dichloro-1,5 anthraquinone à 90,5% (0,0939 mole), 6 g d'oxyde de magnésium (0,15 mole), et 13,6 g d'acétate de sodium (0,165 mole). On chauffe ensuite à 210° C pendant une heure, puis on refroidit à 170° C, ajoute 0,5 g de cuivre en poudre (0,0078 mole) et maintient pendant 4 heures à 170° C jusqu'à disparition de la benzamido-1 amino-4 anthraquinone en CCM.

Après dilution par 132,4 g de nitrobenzène et isolement selon l'exemple 1, on obtient 24,83 g de benzamido-4 chloro-5' dianthrimide-1,1' de pureté 74%, soit un rendement chimique de 73,9% par rapport à la benzamido-1 amino-4 anthraquinone introduite.

Les caractéristiques analytiques du produit recristallisé sont identiques à celles du produit de l'exemple 4.

## Exemple 8

Dans un autoclave de 1 500 ml on charge 600 g d'eau distillée, 60,7 g de dichloro-1,5 anthraquinone à 91% (0,1994 mole), 72,4 g de benzamido-1 amino-4 anthraquinone à 85,5% (0,181 mole), 40 g de carbonate de magnésium (0,475 mole) et 10 g d'acétate cuivrique monohydraté (0,0501 mole). On chauffe ensuite en 4 heures jusqu'à 210° C et maintient à cette température pendant 6 heures. Après refroidissement à 100° C, la masse réactionnelle est essorée et lavée à l'eau. On réempâte le gâteau dans 800 g d'eau et 115,2 g d'acide chlorhydrique 30%, puis chauffe à 95° C pendant 2 heures. Après isolement par essorage, lavage à l'eau distillée jusqu'à neutralité et séchage, on obtient 119,5 g de benzamido-4 chloro-5' dianthrimide-1,1' de pureté 67%, soit un rendement chimique de 75,95% par rapport à la benzamido-1 amino-4 anthraquinone introduite.

Les caractéristiques analytiques du produit recristallisé sont identiques à celles du produit de l'exemple 4.

## Exemple 9

On opère comme à l'exemple 3, mais en remplaçant, d'une part, la benzamido-1 amino-4 anthraquinone par la même quantité de benzamido-1 amino-5 anthraquinone à 99% et, d'autre part, la dichloro-1,4 anthraquinone par le même quantité de dichloro-1,5 anthraquinone à 90,5% (0,0939 mole). Après isolement, on obtient 22,5 g d'un produit dont la teneur en benzamido-5 chloro-5' dianthrimide-1,1'

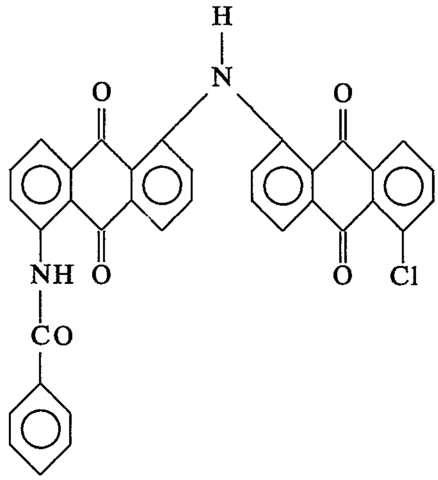

déterminée par HPLC est de 94,6%, soit un rendement chimique de 73,8% par rapport à la benzamido-1 amino-5 anthraquinone introduite.

Après recristallisation de ce produit brut selon le mode opératoire décrit à l'exemple 1, on obtient 19,8 g de benzamido-5 chloro-5' dianthrimide-1,1' à 97,9% de pureté (HPLC), présentant les caractéristiques suivantes:

Analyse élémentaire (%):
Calculé pour $C_{35}H_{19}ClN_2O_5$
C 72,10 H 3,26 Cl 6,09 N 4,80 O 13,73
Trouvé
C 71,40 H 3,28 Cl 5,45 N 4,73 O 13,99

Spectre de masse:
pic unique pour $M^+$ à m/e = 582.

## Exemple 10

On opère comme à l'exemple 3, mais en utilisant 20 g de benzamido-1 amino-4 anthraquinone à 85,5% (0,05 mole) et en remplaçant la dichloro-1,4 anthraquinone par 28,55 g de dichloro-1,8 anthraquinone à 94,3% (0,0972 mole). Après isolement, on obtient 16,97 g de benzamido-4 chloro-8' dianthrimide-1,1' brut

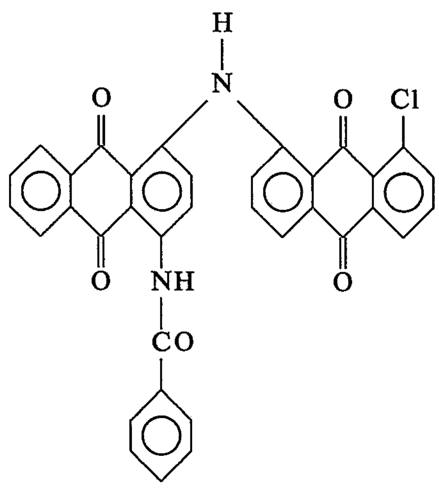

qui, après recristallisation comme à l'exemple 1, fournit 13,6 g de benzamido-4 chloro-8 dianthrimide-1,1' purifié présentant les caractéristiques suivantes:

Analyse élémentaire (%):
Calculé pour $C_{35}H_{19}ClN_2O_5$
C 72,10 H 3,26 Cl 6,09 N 4,80 O 13,73

Trouvé
C 71,49 H 3,38 Cl 5,3 N 4,96 O 14,28

Spectre de masse:
$M^+$ à m/e = 582 (autres pics à 888, 564 et 548).

## Exemple 11

On opère comme à l'exemple 3 à partir de 90,3 g de nitrobenzène, 18 g de benzamido-1 amino-5 anthraquinone à 92% (0,0484 mole), 28,55 g de dichloro-1,8 anthraquinone à 94,3% (0,0972 mole), 15,1 g de bicarbonate de potassium (0,151 mole) et 0,7 g d'acétate cuivrique monohydraté (0,0035 mole). Après isolement on obtient 12 g de benzamido-5 chloro-8' dianthrimide-1,1' brut

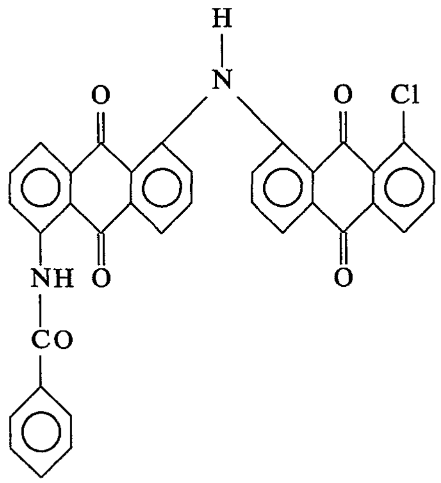

qui, après recristallisation selon l'exemple 1, fournit 9 g de benzamido-5 chloro-8' dianthrimide-1,1' purifié présentant les caractéristiques suivantes:

Analyse élémentaire (%):
Calculé pour $C_{35}H_{19}ClN_2O_5$
C 72,10 H 3,26 Cl 6,09 N 4,80 O 13,73
Trouvé
C 70,71 H 3,46 Cl 5,40 N 4,82 O 14,31

Spectre de masse:
$M^+$ à m/e = 582 (autres pics à 564 et 548).

## Exemple 12

On opère comme à l'exemple 3 à partir de 32,5 g de nitrobenzène, 6 g de benzamido-1 amino-8 anthraquinone à 85% (0,0149 mole), 10 g de dichloro-1,5 anthraquinone à 90,5% (0,0326 mole), 4,5 g de bicarbonate de potassium (0,045 mole) et 0,25 g d'acétate cuivrique monohydraté (0,0012 mole). Après isolement on obtient 5 g de benzamido-8 chloro-5' dianthrimide-1,1' brut

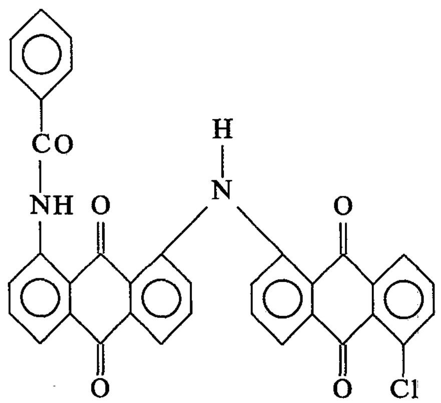

qui, après recristallisation selon le mode opératoire, de l'exemple 1, fournit 3,95 g de benzamido-8 chloro-5' dianthrimide-1,1' purifié présentant les caractéristiques suivantes:

Analyse élémentaire (%):
Calculé pour $C_{35}H_{19}ClN_2O_5$
C 72,10 H 3,26 Cl 6,09 N 4,80 O 13,73
Trouvé
C 70,84 H 3,41 Cl 5,87 N 4,87 O 14,37

Spectre de masse:
$M^+$ à m/e = 582 (autres pics à 564, 548, 458 et 424).

Exemple 13

a) Benzamidation du benzamido-4 bromo-5' dianthrimide-1,1'

On mélange 120,4 g de nitrobenzène et 12,5 g de benzamido-4 bromo-5' dianthrimide-1,1' à 85% (0,0169 mole) tel qu'obtenu à l'exemple 1. On chauffe à 120° C pendant 30 minutes, puis ajoute 3,63 g de benzamide à 98% (0,03 mole), 5 g de bicarbonate de sodium (0,0595 mole) et 0,4 g d'iodure cuivreux (0,0021 mole), et chauffe à 165° C pendant 9 heures jusqu'à disparition du benzamido-4 bromo-5' dianthrimide-1,1' en CCM.

La masse réactionnelle est ensuite distillée sous vide jusqu'à élimination du nitrobenzène, puis le produit est broyé et réempâté dans 125 g d'eau à 80° C. On ajoute ensuite lentement 14,4 g d'acide chlorhydrique à 30%, maintient pendant trois heures à 80° C, puis essore à 80° C, lave à l'eau distillée jusqu'à neutralité et sèche.

On obtient ainsi 12 g d'un produit dont la teneur en dibenzamido-4,5' dianthrimide-1,1'

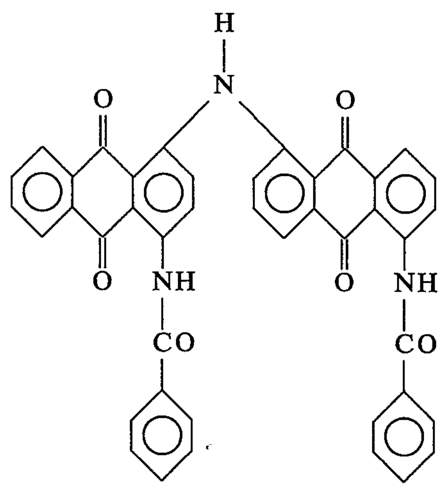

déterminée par HPLC est de 62,5%.

Cette benzamidation peut également être réalisée directement sur le produit obtenu selon le premier paragraphe de l'exemple 1, c'est-à-dire sans isolement du benzamido-4 bromo-5' dianthrimide-1,1'.

b) Cyclisation — Oxydation du dibenzamido-4,5' dianthrimide-1,1'

A une solution de 92 g d'acide sulfurique à 98% et 0,25 g de sulfate ferreux, refroidie à 12° C, on ajoute lentement sans dépasser 15° C 10 g du dibenzamido-4,5' dianthrimide-1,1' à 62,5% obtenu ci-dessus. On maintient pendant 2 heures à 12—15° C, puis verse le mélange dans une solution de 0,3 g de ricinoléate de butyle sulfate (sel de sodium) dans 300 g d'eau. On ajoute ensuite 5 g de chlorate de sodium, porte à 95° C et maintient à cette température pendant 3 heures, puis à 100° C pendant encore 3 heures.

Après essorage à 100° C, lavage à l'eau distillée jusqu'à neutralité et séchage, on obtient 8 g de colorant VAT BROWN 3.

Exemple 14

Dans un autoclave de 1 500 ml on charge 500 ml d'eau distillée, 69,6 g de benzamido-1 amino-4 anthraquinone à 85,5% (0,174 mole), 53,23 g de dichloro-1,5 anthraquinone à 94,7% (0,182 mole), 13,92 g d'oxyde de magnésium (0,348 mole), 7,5 g d'acétate cuivrique monohydraté (0,0375 mole) et 1 g de (hydroxy-2' méthyl-3' benzyl)-1 hydroxy-2 naphtalène-sulfonate de sodium (dispersant). On chauffe ensuite à 190° C durant 8 heures (pression maximale atteinte: 16,5 bars). Après refroidissement à 100° C, la masse réactionnelle est essorée et lavée à l'eau. Un échantillon du gâteau de filtration séché révèle la disparition totale de la benzamido-1 amino-4 anthraquinone en CCM.

On réempâte le gâteau dans 800 g d'eau et 115,2 g d'acide chlorhydrique à 30%, puis chauffe à 95° C pendant 2 heures. Après isolement par essorage et lavage à l'eau distillée jusqu'à neutralité, on isole 109,9 g de benzamido-4 chloro-5' dianthrimide de pureté 69,3%, soit un rendement chimique de 75,1% par rapport à la benzamido-1 amino-4 anthraquinone introduite.

Exemple 15

Dans 120,4 g de nitrobenzène on introduit 18,75 g de p. méthylbenzamido-1 amino-4 anthraquinone à 95% (0,05 mole), 28,85 g de dichloro-1,5 anthraquinone à 96% (0,1 mole), 12,6 g de bicarbonate de sodium (0,15 mole) et 0,7 g d'acétate cuivrique monohydrate (0,0035 mole), puis chauffe à 160°C pendant 4 heures jusqu'à disparition de la p. méthylbenzamido-1 amino-4 anthraquinone en CCM.

Après dilution avec 180,6 g de nitrobenzène, on chauffe le mélange réactionnel à 160° C durant une heure et essore puis lave à cette température selon l'exemple 1. On obtient ainsi 18,2 g de p. méthylbenzamido-4 chloro-5' dianthrimide-1,1' brut

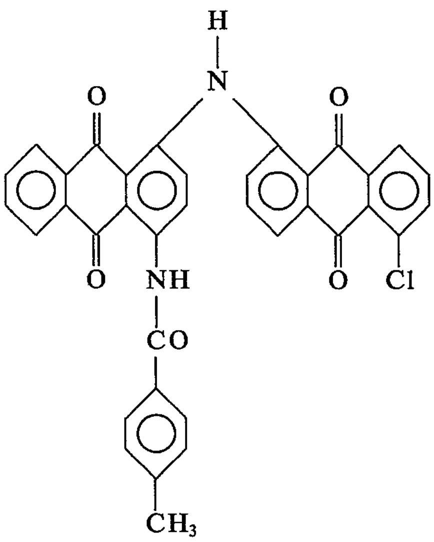

qui, après recristallisation selon l'exemple 1, fournit 11,85 g de p. méthylbenzamido-4 chloro-5' dianthrimide-1,1' purifié présentant les caractéristiques suivantes:

Analyse élémentaire (%):

Calculé pour $C_{36}H_{21}ClN_2O_5$

| | | | | |
|---|---|---|---|---|
| C 72,42 | H 3,52 | Cl 5,95 | N 4,69 | O 13,41 |

Trouvé

| | | | | |
|---|---|---|---|---|
| C 72,35 | H 3,64 | Cl 5,42 | N 4,63 | O 14,07 |

Exemple 16

Dans 252,85 g de nitrobenzène on introduit 49,85 g de dichloro-1,5 anthraquinone 100% (0,18 mole), 8,8 g de bicarbonate de sodium (0,10 mole) et 0,5 g d'acétate cuivrique monohydraté (0,0025 mole), puis chauffe à 160°C et introduit en 3 heures 14,16 g de (phtaloyl[c]benzamido)-4 amino-1 anthraquinone (préparée par condensation d'une mole de chlorure d'acide anthraquinone-carboxylique-2 sur la diamino-1,4 anthraquinone, et dont la CCM fait apparaître une tache unique), (0,03 mole). Après 5 heures supplémentaires à 160° C, le mélange réactionnel est porté durant deux heures à 170° C jusqu'à disparition en CCM de la (phtaloyl[c]benzamido)-4 amino-1 anthraquinone.

Le mélange réactionnel est ensuite refroidi à 160° C, puis essoré et lavé selon l'exemple 1. On obtient

18,45 g de (phtaloyl[c]benzamido)-4 chloro-5′ dianthrimide-1,1′

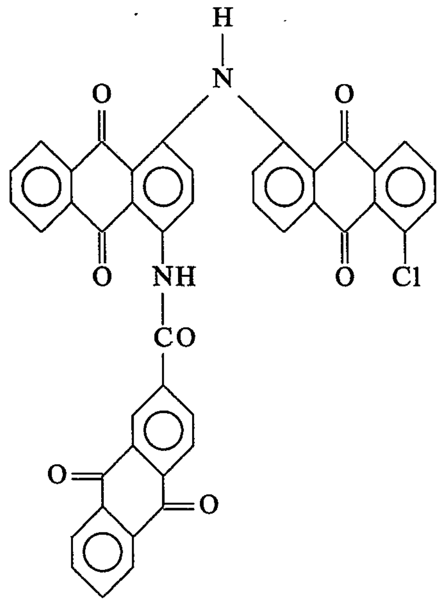

dont l'analyse élémentaire (%) est la suivante:

Calculé pour $C_{43}H_{21}ClN_2O_7$

| | | | | | |
|---|---|---|---|---|---|
| C 72,42 | H 2,94 | Cl 4,98 | N 3,93 | O 15,72 | Cendres 0 |

Trouvé

| | | | | | |
|---|---|---|---|---|---|
| C 69,75 | H 3,05 | Cl 4,62 | N 3,67 | O 15,80 | Cendres 2,4 |

## Exemple 17

### a) Benzamidation du benzamido-5 chloro-5′ dianthrimide-1,1′

A un mélange de 240,8 g de nitrobenzène et 23,3 g de benzamido-5 chloro-5′ dianthrimide-1,1′ à 97,9% identique à celui obtenu dans l'exemple 9 (0,039 mole), on ajoute 9,68 g de benzamide à 98% (0,08 mole), 11,76 g de bicarbonate de sodium (0,14 mole), 1,15 g iodure cuivreux (0,006 mole) et 10 g hexaméthyl-phosphorotriamide (0,0558 mole). On chauffe ensuite pendant 11 heures à 180°C puis 5 heures à 190°C jusqu'à disparition du benzamido-5 chloro-5′ dianthrimide-1,1′ en CCM.

Après refroidissement du mélange réactionnel à 140°C et filtration à 140°C sur filtre à plaque de verre fritté de porosité n° 2, lavage du précipité avec 50 g de nitrobenzène à 140°C, puis au méthanol et à l'eau jusqu'à neutralité, on obtient 22,2 g de dibenzamido-5,5′ dianthrimide-1,1′

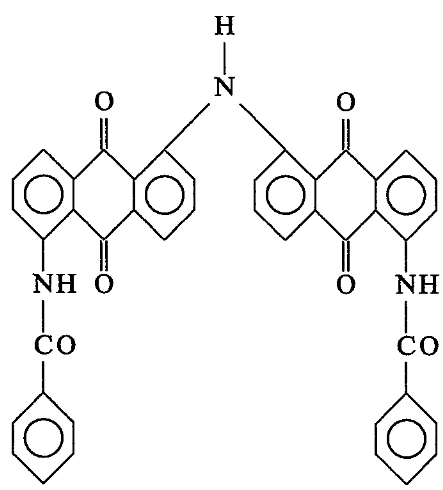

dont la teneur déterminée par HPLC est de 85,7%. Rendement chimique: 72,2%.

b) Cyclisation — Oxydation du dibenzamido-5,5′ dianthrimide-1,1′

A une solution de 80 g d'acide sulfurique à 99%, refroidie à 15° C, on ajoute lentement sans dépasser 15° C, 10 g de dibenzamido-5,5′ dianthrimide-1,1′ à 85,7% obtenu ci-dessus, puis 0,85 g de nitrite de sodium et maintient pendant 2 heures à 15° C. Ensuite, on dilue le mélange réactionnel par 56,7 g d'acide sulfurique ajouté en 2 heures sans dépasser 15° C.

La masse réactionnelle est alors coulée sur un mélange de 166,7 g d'eau et 100 g de glace. Après essorage du précipité et lavage jusqu'à neutralité, la pâte de presse obtenue est réempâtée avec 50 g d'eau et 3,4 g de lessive de soude à 30%. Ce mélange est chauffé à 85° C, puis on introduit 0,05 g de chlorure ferrique et 200 ml d'eau de javel à 45° chlorométrique en trois fractions en l'espace d'environ 8 heures.

Après filtration à 85° C et lavage à l'eau puis par 200 ml d'acide chlorhydrique 5% suivi d'un lavage à l'eau jusqu'à neutralité, on obtient 7,4 g de colorant VAT ORANGE 15.

Exemple 18

Condensation suivie d'une benzamidation in situ.

Dans 500 g de nitrobenzène on introduit 85,5 g de amino-1 benzamido-4 anthraquinone à 85,5% (0,2137 mole) et 87,44 g de dichloro-1,5 anthraquinone à 94,7% (0,2989 mole). Après chauffage à 120° C on ajoute 63 g de bicarbonate de sodium (0,75 mole) et 3,5 g d'acétate de cuivre monohydraté (0,01755 mole), puis on chauffe à 160° C pendant 7 heures jusqu'à disparition de l'amino-1 benzamido-4 anthraquinone en CCM.

Après dilution lente par 554 g de nitrobenzène en maintenant la température au-dessus de 140° C et maintient durant 2 heures à 160° C, on ajoute 62,1 g de benzamide à 98% (0,503 mole), 63 g de bicarbonate de sodium (0,75 mole), 4,75 g de iodure cuivreux (0,02494 mole) et 27,7 g hexaméthyl phosphorotriamide (0,1546 mole), puis on chauffe à 180° C et maintient 10 heures à cette température jusqu'à disparition en CCM du chloro-5′ benzamido-4 dianthrimide.

La masse réactionnelle est alors distillée sous vide sans dépasser 160° C; le résidu solide est ensuite broyé et traité par 2 litres d'eau à 90° C. Après essorage et lavage jusqu'à neutralité on obtient 195,5 g de dibenzamido-4,5′ dianthrimide à 47,2% (HPLC).
Rendement chimique: 64,7%.

**Revendications**

1. Procédé pour la préparation des $\alpha$-benzamido $\alpha'$-halogéno dianthrimides-1,1′ de formule générale:

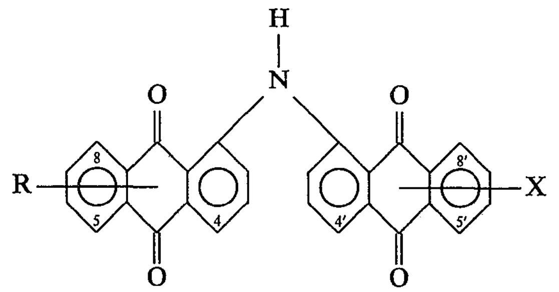

(I)

dans laquelle R représente un groupe benzamido éventuellement substitué, fixé en position 4, 5 ou 8 et X représente un atome d'halogène fixé en position 4′, 5′ ou 8′, caractérisé en ce que l'on fait réagir une amino-benzamido-anthraquinone de formule:

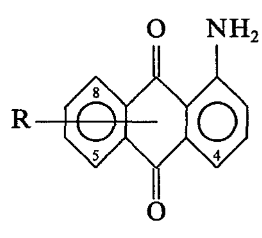

(II)

avec une dihalogéno-anthraquinone de formule:

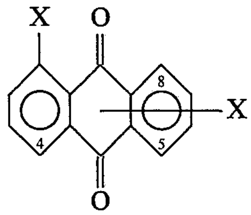

(III)

dans l'eau ou dans un solvant organique, à une température comprise entre 130°C et 220°C, en présence de 0,01 à 0,3 mole d'un catalyseur à base de cuivre par mole d'amino-benzamido-anthraquinone et de 1,5 à 7,5 moles d'un agent capteur d'acide par mole d'amino-benzamido-anthraquinone, le rapport molaire: dihalogéno-anthraquinone/amino-benzamido-anthraquinone étant supérieur à 1.

2. Procédé selon la revendication 1 dans lequel le catalyseur est le cuivre ou un sel de cuivre.

3. Procédé selon la revendication 1 ou 2 dans lequel l'agent capteur d'acide est un sel alcalin ou alcalino-terreux ou un oxyde alcalino-terreux.

4. Procédé selon l'une des revendications 1 à 3 dans lequel on opère dans l'eau à une température comprise entre 185 et 220°C.

5. Procédé selon l'une des revendications 1 à 3 dans lequel on opère au sein d'un solvant organique à une température comprise entre 140 et 170°C avec un rapport molaire dihalogéno-anthraquinone/amino-benzamido-anthraquinone au moins égal à 1,35.

6. Procédé selon la revendication 5 dans lequel le solvant organique est le nitrobenzène, le naphtalène le nitro-2 toluène ou un polyhalogénobenzène.

7. Procédé selon l'une des revendications 1 à 6 dans lequel la concentration molaire initiale en amino-benzamido-anthraquinone dans le milieu réactionnel est supérieure à 0,1 mole/litre.

8. Les $\alpha$-benzamido halogéno-5' ou 8' dianthrimides-1,1'de formule générale:

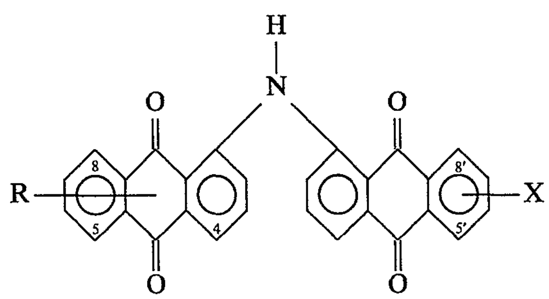

dans laquelle R représente un groupe benzamido éventuellement substitué, fixé en position 4, 5 ou 8 et X représente un atome d'halogène fixé en position 5' ou 8' (position 4' exclue).

9. Dianthrimides selon la revendication 8 dans lesquels les substituants R et X sont fixés respectivement en positions 4 et 5' ou 5 et 5'.

10. Dianthrimides selon la revendication 8 ou 9 dans lesquels X représente un atome de brome ou de chlore et R représente le groupe benzamido non substitué.

11. L'application des dianthrimides obtenus selon l'une des revendications 1 à 7 à la fabrication de colorants de cuve du type $\alpha,\alpha'$-dibenzamido diphtaloyl carbazole.

12. Application selon la revendication 11 qui consiste à soumettre un dianthrimide obtenu selon l'une des revendications 1 à 7 à une benzamidation et à une cyclisation.

**Patentansprüche**

1. Verfahren zur Herstellung von $\alpha$-Benzamido-$\alpha'$-halogen-1,1'-dianthrimiden der allgemeinen Formel I

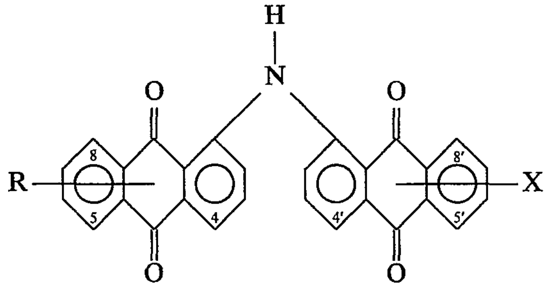

(I)

worin R einen gegebenenfalls substituierten Benzamidrest, der in Position 4, 5 oder 8 fixiert ist, bedeutet und X ein in Position 4', 5' oder 8' fixiertes Halogenatom darstellt, dadurch gekennzeichnet, daß ein Amino-benzamido-anthrachinon der allgemeinen Formel II

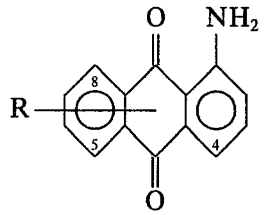

(II)

mit einem Dihalogenanthrachinon der allgemeinen Formel III

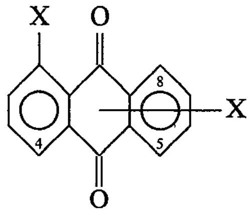

(III)

in Wasser oder einem organischen Lösungsmittel bei einer Temperatur im Bereich von 130° C bis 220° C in Anwesenheit von 0,01 bis 0,3 Mol eines Katalysators auf Kupferbasis pro Mol Amino-benzamido-anthrachinon und von 1,5 bis 7,5 Mol eines Säureabfangmittels pro Mol Amino-benzamido-anthrachinon, wobei das molare Verhältnis Dihalogen-anthrachinon/Amino-benzamido-anthrachinon größer als 1 ist, umgesetzt wird.

2. Verfahren nach Anspruch 1, dadurch gekennzeichnet, daß der Katalysator Kupfer oder ein Kupfersalz ist.

3. Verfahren nach Anspruch 1 oder Anspruch 2, dadurch gekennzeichnet, daß das Säureabfangmittel ein Alkalimetallsalz oder ein Erdalkalimetallsalz oder ein Erdalkalimetalloxid ist.

4. Verfahren nach einem der Ansprüche 1 bis 3, dadurch gekennzeichnet, daß es in Wasser unter einer Temperatur von 185 bis 220° C durchgeführt wird.

5. Verfahren nach einem der Ansprüche 1 bis 3, dadurch gekennzeichnet, daß es in einem organischen Lösungsmittel unter einer Temperatur von 140 bis 170° C bei einem molaren Verhältnis Dihalogen-anthrachinon/Amino-benzamido-anthrachinon von wenigstens 1,35 durchgeführt wird.

6. Verfahren nach Anspruch 5, dadurch gekennzeichnet, daß das organische Lösungsmittel Nitrobenzol, Naphthalin, 2-Nitrotoluol oder ein Polyhalogenbenzol ist.

7. Verfahren nach einem der Ansprüche 1 bis 6, dadurch gekennzeichnet, daß die anfängliche molare Konzentration von Amino-benzamido-anthrachinon im Reaktionsmedium höher als 0,1 Mol/Liter liegt.

8. α-Benzamido-5'-halogen- oder -8'-halogen-1,1'-dianthrimide der allgemeinen Formel

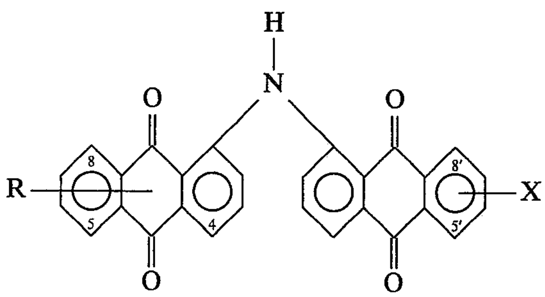

worin R einen gegebenenfalls substituierten Benzamidorest in Position 4, 5 oder 8 darstellt und X ein Halogenatom in Position 5' oder 8' bedeutet (wobei Position 4' ausgeschlossen ist).

9. Dianthrimide nach Anspruch 8, dadurch gekennzeichnet, daß die Substituenten R und X in den Positionen 4 und 5' oder 5 und 5' fixiert sind.

10. Dianthrimide nach Anspruch 8 oder Anspruch 9, dadurch gekennzeichnet, daß X ein Bromatom oder Chloratom bedeutet und R einen nicht substituierten Benzamidorest darstellt.

11. Verwendung der nach einem der Ansprüche 1 bis 7 erhaltenen Dianthrimide bei der Herstellung von Büttenfarbstoffen (colorants de cuve) vom Typ des α,α'-Dibenzamido-diphthaloyl-carbazols.

12. Verwendung nach Anspruch 11, dadurch gekennzeichnet, daß ein Dianthrimid, welches nach einem der Ansprüche 1 bis 7 erhalten worden ist, einer Benzamidierung und Cyclisierung unterworfen wird.

## Claims

1. Process for the preparation of 1,1'-alpha-benzamido-alpha'-halogeno-dianthramides of the general formula:

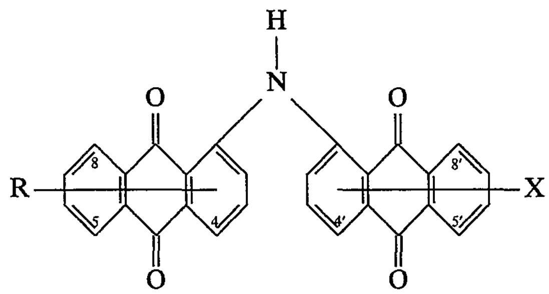

(I)

wherein R represents an optionally substituted benzamido group, attached at the 4, 5 or 8 position and X represents a halogen atom attached at the 4', 5' or 8' position, characterised in that an aminobenzamidoanthraquinone of the formula:

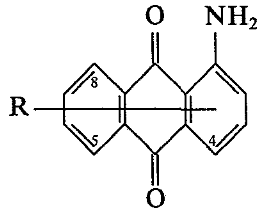

(II)

is reacted with a dihalogenoanthraquinone of the formula:

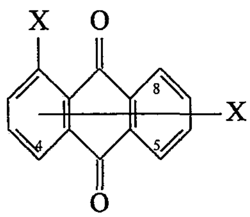

(III)

in water or in an organic solvent, at a temperature between 130° C and 220° C, in the presence of 0.01 to 0.3 mole of a copper-based catalyst per mole of aminobenzamidoanthraquinone and 1.5 to 7.5 mole of an acid binding agent per mole of aminobenzamidoanthraquinone, the molar ratio dihalogenoanthraquinone/aminobenzamidoanthraquinone being greater than 1.

2. Process according to claim 1 in which the catalyst is copper or a copper salt.

3. Process according to claim 1 or 2 in which the acid-binding agent is an alkaline salt or an alkaline-earth salt or an alkaline-earth oxide.

4. Process according to one of the claims 1 to 3 in which the process is performed in water at a temperature between 185° C and 220° C.

5. Process according to one of the claims 1 to 3 in which the process is performed in an organic solvent at a temperature between 140° C and 170° C with a molar ratio of dihalogenoanthraquinone/aminobenzamido-anthraquinone at least equal to 1.35.

6. Process according to claim 5 in which the organic solvent is nitrobenzene, naphthalene, 2-nitrotoluene or a polyhalogenobenzene.

7. Process according to one of the claims 1 to 6 in which the initial molar concentration of aminobenzamidoanthraquinone in the reaction medium is greater than 0.1 mol/litre.

8. 1,1'-Alpha-benzamido-5' or 8'-halogenodianthramides of the general formula:

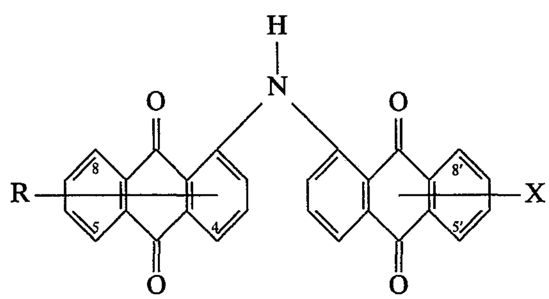

in which R represents an optionally substituted benzamido group, attached at the 4, 5 or 8 position, and X represents a halogen atom attached at the 5′ or 8′ position (4′ position excluded).

9. Dianthramides according to claim 8 in which the substituents R and X are attached at the 4 and 5′ positions or in the 5 and 5′ positions, respectively.

10. Dianthramides according to claim 8 or 9 in which X represents a bromine or chlorine atom and R represents the unsubstituted benzamido group.

11. The application of the dianthramides obtained according to one of the claims 1 to 7 in the production of vat dyes of the alpha,alpha′-dibenzamidodiphthaloylcarbazole type.

12. Application according to claim 11 which consists in subjecting a dianthramide obtained according to one of the claims 1 to 7 to a benzamidation process and a cyclisation process.